# DEMANDE DE BREVET EUROPEEN

(11) **EP 1 354 627 A1**
(43) Date de publication de la demande: **22.10.2003**
(21) Numéro de dépôt: 03290647.1
(22) Date de dépôt: 14.03.2003
(51) Int. Cl.: B01J 31/18, B01J 31/12, B01J 31/14, C07C 2/30

(54) **Complexes organométalliques comportant des ligands phosphonites et leur utilisation pour catalyser l'oligomérisation des oléfines**

(30) Priorité: 29.03.2002 FR 0204107
(71) Demandeur: Institut Français du Pétrole, 92852 Rueil-Malmaison Cedex (FR)
(72) Inventeur: Speiser, Fredy, 67300 Schiltigheim (FR); Braunstein, Pierre, 67000 Strasbourg (FR)

(57) **Abrégé**

Une composition catalytique pour l'oligomérisation des oléfines et en particulier de l'éthylène, comprend le mélange d'au moins un complexe de nickel, préparé par réaction d'un sel de nickel avec un ligand phosphonite, et d'au moins un composé d'hydrocarbylaluminium en faible proportion par rapport au nickel et choisi dans le groupe formé par les composés tris(hydrocarbyl)aluminium, les composés chlorés ou bromés d'hydrocarbylaluminium et les aluminoxanes.

## Description

La présente invention concerne l'oligomérisation des oléfines, en particulier de l'éthylène.

Un objet de l'invention est de fournir une nouvelle composition catalytique. Un autre objet de l'invention est de fournir un procédé d'oligomérisation des oléfines, en particulier de l'éthylène, utilisant cette composition catalytique.

Il est bien connu que les monooléfines-α telles que l'éthylène, le propylène ou le butène-1 peuvent être oligomérisées avec des systèmes catalytiques à base de métaux de transition tels que le nickel, le chrome, le titane, le zirconium ou d'autres métaux, en présence d'un co-catalyseur tel qu'un composé d'hydrocarbylaluminium, un halogénure d'hydrocarbylaluminium ou un aluminoxane. Cependant, beaucoup de catalyseurs de type Ziegler-Natta sont peu actifs lorsque la proportion de co-catalyseur est faible et entraînent une consommation importante de co-catalyseur, puisqu'un rapport molaire aluminium sur métal de transition supérieur à 10 est souvent nécessaire pour obtenir un catalyseur suffisamment actif.

Il a maintenant été trouvé, de façon inattendue, qu'une composition catalytique obtenue en mélangeant au moins un complexe du nickel contenant au moins un ligand de type phosphonite, en combinaison avec au moins un composé d'hydrocarbylaluminium en faible proportion présente une activité améliorée pour l'oligomérisation des oléfines, en particulier de l'éthylène.

Ainsi la composition catalytique de l'invention est définie comme comprenant le mélange :
- d'au moins un complexe de nickel contenant au moins un ligand phosphonite ;
- avec au moins un composé d'hydrocarbylaluminium choisi dans le groupe formé par les composés tris(hydrocarbyl)aluminium et les composés chlorés ou bromés d'hydrocarbylaluminium qui répondent à la formule générale AIR"ₘY₃₋ₘ, dans laquelle R" est un radical hydrocarbyle comprenant de 1 à 6 atomes de carbone, Y est un atome de chlore ou de brome et m est un nombre de 1 à 3, et les aluminoxanes ;
dans un rapport molaire Al/Ni inférieur ou égal à 8/1.

Plus précisément, les complexes de nickel mis en oeuvre dans cette invention ont pour formule générale LNiX₂ dans laquelle L est un ligand phosphonite bi- ou tridenté répondant à la formule générale : 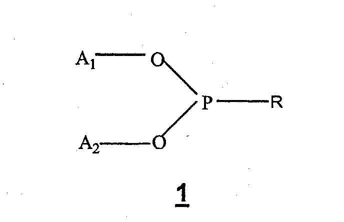 dans laquelle R représente un radical hydrocarboné monovalent contenant jusqu'à 12 atomes de carbone, tel que alkyle, aryle, aralkyle, alkaryle, cycloalkyle ou aryle substitué, et les radicaux A₁-O- et A₂-O-, identiques ou différents, peuvent être choisis parmi les radicaux alkoxy et aryloxy et les radicaux alkoxy portant un hétérocycle azoté, tels que les radicaux alkoxy-pyridine et alkoxy-oxazoline. Plus précisément, les radicaux A₁-O- et A₂-O- peuvent répondre aux formules générales ci-dessous : 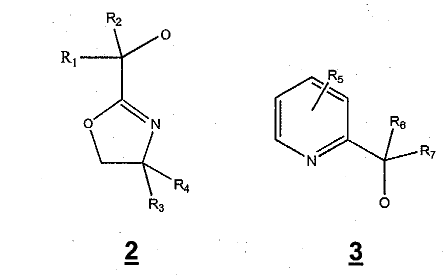 dans lesquelles les radicaux R₁, R₂, R₃, R₄, R₅, R₆ et R₇, qui peuvent être identiques ou différents, sont choisis parmi l'atome d'hydrogène, les radicaux alkyles linéaires ou ramifiés, les radicaux aryles, aralkyles ou alkaryles comportant de 1 à 12 atomes de carbone. Le radical R₅ peut être sur l'une quelconque des positions libres du noyau aromatique. A titre d'exemples non limitatifs, ces substituants peuvent être choisis parmi les radicaux méthyle, éthyle, isopropyle, isobutyle, tert-butyle, cyclohexyle, phényle et benzyle.

Dans la formule 1, les radicaux A₁ et A₂ peuvent être identiques entre eux, comme dans les ligands présentés ci-dessous :

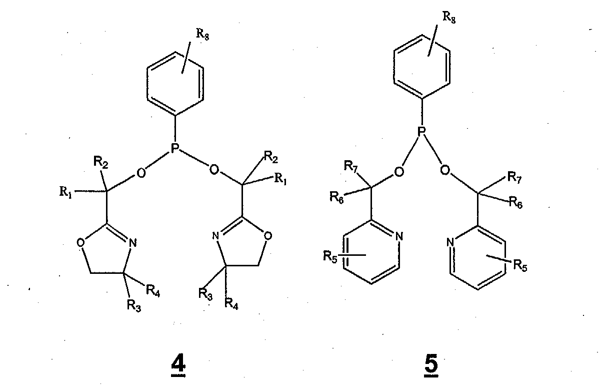

Par ailleurs, dans la formule 1, les radicaux R et A₁, R et A₂ ou A₁ et A₂ peuvent être reliés entre eux et faire partie d'un même radical cyclique, comme par exemple dans les formules présentées ci-dessous :

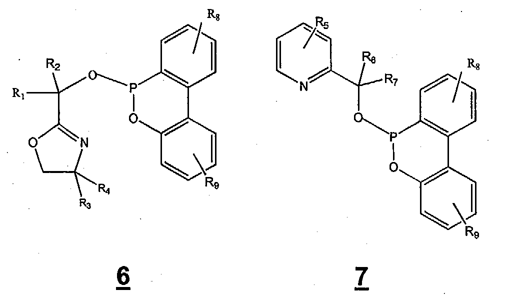

Dans les formules **4** à **7** qui précèdent, les radicaux R₁, R₂, R₃, R₄, R₅, R₆, R₇, R₈ et R₉ sont définis comme les radicaux R₁, R₂, R₃, R₄, R₅, R₆ et R₇ ci-dessus.

Dans la formule du complexe de nickel, X peut être un anion halogénure, Cl, Br ou I, un anion acétylacétonate, acétate, trifluoroacétate ou plus généralement un anion carboxylate R'COO- pour lequel R' est un radical hydrocarbyle, par exemple alkyle, cycloalkyle, alkényle, aryle, aralkyle ou alkaryle contenant jusqu'à 20 atomes de carbone, de préférence un radical hydrocarbyle de 5 à 20 atomes de carbone, éventuellement substitué par des atomes d'halogène (fluor ou chlore). A titre d'exemples non limitatifs, l'anion carboxylate peut être choisi parmi les anions suivants : octoate, éthyl-2 hexanoate, stéarate, oléate, naphténate et adipate.

Les composés d'hydrocarbylaluminium sont choisis dans le groupe formé par les tris(hydrocarbyl)aluminium, les composés chlorés ou bromés d'hydrocarbylaluminium et les aluminoxanes. Les tris(hydrocarbyl)aluminium et les composés chlorés ou bromés d'hydrocarbylaluminium répondent de préférence à la formule générale AIR"ₘY₃₋ₘ dans laquelle R" représente un radical hydrocarboné monovalent contenant par exemple jusqu'à 12 atomes de carbone tel que alkyle, aryle, aralkyle, alkaryle ou cycloalkyle, Y représente un atome d'halogène choisi par exemple parmi le chlore et le brome, Y étant de préférence un atome de chlore, m prend une valeur de 1 à 3, m étant de préférence égal à 1. Comme exemples de tels composés de formule AIR"ₘY₃₋ₘ, on peut mentionner le sesquichlorure d'éthylaluminium, le dichloroéthylaluminium, le dichloroisobutylaluminium et le chlorodiéthylaluminium et le triéthylaluminium.

La préparation des ligands de type phosphonite est effectuée selon les méthodes décrites dans la littérature (P. Braunstein et al. Organometallics 2000, 19, 2676-2683).

La préparation du complexe de nickel LNiX₂ se fait selon les méthodes connues dans la littérature pour la synthèse de complexes du nickel avec un ligand neutre. Tout procédé de préparation de ce composé peut convenir, comme par exemple la réaction du ligand phosphonite avec un sel de nickel dans un solvant organique, par exemple un éther, un alcool, un solvant chloré, tel que le dichlorométhane. Le complexe peut être préparé *in situ* dans le solvant utilisé pour la réaction d'oligomérisation. Dans ce cas, l'ordre de mélange du sel de nickel et du ligand phosphonite n'est pas critique. Cependant, on préfère préparer d'abord une solution d'un sel de nickel soluble en milieu organique, comme par exemple un carboxylate de nickel et ajouter ensuite le ligand phosphonite

Le complexe du nickel et le co-catalyseur d'aluminium peuvent être mis en contact dans un solvant constitué par un hydrocarbure saturé comme l'hexane, le cyclohexane, l'heptane, le butane ou l'isobutane, par un hydrocarbure insaturé comme une monooléfine ou une dioléfine comportant par exemple de 4 à 20 atomes de carbone, ou par un hydrocarbure aromatique tel que le benzène, le toluène, l'ortho-xylène, le mésitylène, l'éthylbenzène ou le chlorobenzène, purs ou en mélange.

La concentration du nickel dans la solution catalytique varie en général de 1.10⁻⁵ à 0,1 mole/L, de préférence de 5.10⁻⁵ à 1.10⁻² mole/L.

Le rapport molaire entre l'hydrocarbylaluminium et le complexe de nickel est choisi entre 1/1 à 8/1, de préférence de 1/1 à 6/1.

L'ordre de mélange des deux constituants de la composition catalytique n'est pas critique. Cependant, on préfère ajouter le composé d'hydrocarbylaluminium sur la solution du complexe.

La réaction d'oligomérisation de l'éthylène peut être effectuée sous une pression totale de 0,5 à 15 MPa, de préférence de 1 à 8 MPa, et à une température de 20 à 180 °C, de préférence de 40 à 140 °C.

Dans un mode particulier de mise en oeuvre de la réaction catalytique d'oligomérisation en discontinu, on introduit un volume choisi de la solution catalytique, constituée comme décrit ci-dessus, dans un réacteur muni des dispositifs habituels d'agitation, de chauffage et de refroidissement, puis on pressurise par de l'éthylène à la pression désirée, et on ajuste la température à la valeur souhaitée. Le réacteur d'oligomérisation est maintenu à pression constante par introduction d'éthylène jusqu'à ce que le volume total de liquide produit représente, par exemple, de 2 à 50 fois le volume de la solution catalytique primitivement introduit. On détruit alors le catalyseur par tout moyen habituel connu de l'homme du métier, puis on soutire et on sépare les produits de la réaction et le solvant.

En cas d'opération en continu, la mise en oeuvre est par exemple la suivante : la solution catalytique est injectée en même temps que l'éthylène dans un réacteur agité par les moyens mécaniques classiques ou par une recirculation extérieure, et maintenu à la température souhaitée. On peut aussi injecter séparément les composants du catalyseur dans le milieu réactionnel, par exemple le complexe du nickel comportant le ligand phosphonite d'une part, et le composé d'hydrocarbylaluminium, d'autre part. L'éthylène est introduit par une vanne d'admission asservie à la pression, qui maintient celle-ci constante. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits de la réaction ainsi que le solvant sont séparés, par exemple par distillation. L'éthylène qui n'a pas été transformé peut être recyclé dans le réacteur.

Les exemples suivants illustrent l'invention sans en limiter la portée.

### EXEMPLE 1

### a) Préparation du complexe (NOPON)NiCl₂

Le ligand phosphonite **4** pour lequel R1 = R2 = R3 = R4 = méthyle et R8 = H, nommé NOPON en abrégé, a été préparé selon les méthodes décrites dans la littérature (P. Braunstein et al. Organometallics 2000, 19, 2676-2683).

Le ligand phosphonite **4** (0,420 g, 0,998 mmole) est mis en solution dans 30 mL de tétrahydrofuranne (THF), puis, après addition d'un équivalent de chlorure de nickel-diméthyléther, NiCl₂(DME) (0,217 g, 0,998 mmole), la solution est agitée pendant 24 heures. Après évaporation du solvant, le complexe vert est repris dans du dichlorométhane, la solution est filtrée sur célite, puis concentrée. Le solide vert est séché sous vide. Rendement : 0,660 g, soit 75 %. Ce produit est caractérisé par une bande d'absorption Infra-Rouge à 1620 cm⁻¹, caractéris tique de la double liaison C=N coordinée au nickel.

### b) Oligomérisation de l'éthylène

Dans un ballon en verre de 50 mL placé sous atmosphère inerte, on introduit à l'abri de l'air et de l'humidité 0,1.10⁻³ mole de complexe de nickel, que l'on dilue avec 25 mL de toluène distillé et que l'on conserve sous atmosphère inerte. Dans un autoclave en acier inoxydable d'un volume utile de 100 mL, muni d'une double enveloppe permettant de réguler la température par circulation d'huile, on introduit, dans l'ordre, sous atmosphère d'éthylène, et à la température ambiante, 10 mL de la solution de nickel préparée ci-dessus, soit 0,04.10⁻³ mole de nickel, et 0,08.10⁻³ mole de dichloroéthylaluminium en solution dans 5 mL de toluène. La température est alors portée à 30 °C et la pression d'éthylène est maintenue à 1 MPa.

Après 70 minutes de réaction, l'introduction d'éthylène est arrêtée et le réacteur est refroidi et dégazé, puis le gaz et le liquide qui a été soutiré au moyen d'une seringue sont analysés par chromatographie en phase vapeur. On a consommé 35 g d'éthylène en 70 minutes. La composition des produits est donnée dans le Tableau 1. Le facteur de croissance de la distribution géométrique de Schulz-Flory (SF) est représenté par k_{α}.

### EXEMPLE 2

Dans le même appareillage que celui qui a été utilisé pour l'Exemple 1 et dans les mêmes conditions, à ceci près que le rapport dichloroéthylaluminium sur nickel est de 6/1 au lieu de 2/1, on a consommé 41 g d'éthylène en 70 minutes de réaction. La composition des produits est donnée dans le Tableau 1.

### EXEMPLE 3

### a) Préparation du complexe de nickel

Pour la synthèse du complexe, on opère comme dans l'Exemple 1 à ceci près que le ligand utilisé pour complexer le nickel a pour formule **7** avec R5 = H, R6 = R7 = méthyle, R8 = R9 = H.

Synthèse du ligand **7** : Les produits de départ sont préparés selon des modes opératoires publiés: S.D. Pastor et al. "Phosphorus and Sulfur" 1987, vol. 31, p. 71, pour le 6-chloro-6H-dibenz[c,e] [1,2] oxaphosphorine, et D.S. Noyce et al. J. Org . Chem 1973, 38, 2260, pour le 2-(2-pyridyl)-2-propanol.

Une solution de 2-(2-pyridyl)-2-propanol (0,580 g, 4,26 mmole) dans 30 mL de tétrahydrofuranne est refroidie à -78 °C, puis agitée une heure après addition d'un équivalent de butyllithium. On lui ajoute ensuite, goutte à goutte, une solution de 6-chloro-6H-dibenz[c,e] [1,2] oxaphosphorine (1 g, 4,26 mmole) en solution dans 20 mL de tétrahydrofuranne et on laisse le mélange revenir lentement à 20 °C sous agitation pendant environ 15 heures. Après hydrolyse avec de l'eau dégazée, extraction à l'éther, séchage de la phase organique sur MgSO₄ et évaporation du solvant, on obtient 1,16 g d'huile jaune soit un rendement de 80%.

Ce produit a été caractérisé par ses spectres de résonance magnétique du proton et du phosphore :

| | |
|---|---|
| ¹H-NMR(CDCl₃) δ(ppm) | 1,74 (3H, C(CH₃)₂), 1,76 (3H, C(CH₃)₂), 7,17 - 7,35 (m, 4 H, OPh), 7,65 (m, 1H, py-H₄), 7,44 (m, 1H, py-H₅), 7,60 (m, 1H, py-H₃), 7,43 - 8,04 (m, 4H, PPh), 8,04 (t, 1H, py-H₄, ³*J* (H,H) = 6,0 Hz), 9,2 (d, 1H, py-H₆, ³*J* (H,H) = 8,1 Hz) |
| ³¹P-NMR (CDCl₃) δ (ppm) | 119,8 |

### b) Oligomérisation de l'éthylène

On opère dans le même appareillage que celui qui a été utilisé pour l'Exemple 1 et dans les mêmes conditions. Le rapport dichloroéthylaluminium sur nickel est de 2/1. La composition des produits est donnée dans le Tableau 1.

### EXEMPLE 4

On opère comme dans l'Exemple 3, à ceci près que le rapport dichloroéthylaluminium sur nickel est de 6/1 au lieu de 2/1.

### EXEMPLE 5

### a) Préparation du complexe de nickel

On opère comme dans l'Exemple 1, à ceci près que le ligand utilisé pour complexer le nickel a pour formule **6** avec R1 = R2 = R3 = R4 = méthyle, R8 = R9 = H.

La synthèse du ligand **6** est effectuée avec un rendement de 75 % selon les méthodes décrites dans la littérature par réaction du 4,4'-diméthyl-2-(1-hydroxy-1-méthyléthyl)-4,5-dihydrooxazole sur le 6-chloro-6H-dibenz[c,e] [1,2] oxaphosphorine en présence de triéthylamine (voir références ci-dessus).

| | |
|---|---|
| ¹H-NMR (CD₂Cl₂) δ (ppm) | 1,30 (6H), 1,38 (3H) ;1,62 (3H) ; 4,01 (2 H), 7,1 - 7,7 (m, 6H), 7,98 (m, 2H). |
| ³¹P-NMR (CD₂Cl₂) δ(ppm) | 120,9 |

### b) Oligomérisation de l'éthylène

On opère dans le même appareillage que celui qui a été utilisé pour l'Exemple 1 et dans les mêmes conditions. Le rapport dichloroéthylaluminium sur nickel est de 2/1. La composition des produits est donnée dans le Tableau 1.

**Tableau 1**

| Exemple | Répartition des oligomères (% poids) | | | | k_{α} (SF) | Productivité (g d'éthylène/g de Ni /h) |
|---|---|---|---|---|---|---|
| | C4 | C6 | C8 | C10+ | | |
| 1 | 75,8 | 22,2 | 1,8 | 0,2 | 0,14 | 12 700 |
| 2 | 70,4 | 26,8 | 2,6 | 0,2 | 0,16 | 15 000 |
| 3 | 81,4 | 17,0 | 1,4 | 0,2 | 0,11 | 21 100 |
| 4 | 74,8 | 22,5 | 2,5 | 0,2 | 0,15 | 27 400 |
| 5 | 72,9 | 25,5 | 1,5 | 0,1 | 0,17 | 21 200 |

## Revendications

1. Composition catalytique comprenant le mélange :
- d'au moins un complexe de nickel contenant au moins un ligand phosphonite ;
- avec au moins un composé d'hydrocarbylaluminium choisi dans le groupe formé par les composés tris(hydrocarbyl)aluminium, les composés chlorés ou bromés d'hydrocarbylaluminium répondant à la formule générale AIR"ₘY₃₋ₘ, dans laquelle R" est un radical hydrocarbyle comprenant de 1 à 6 atomes de carbone, Y est un atome de chlore ou de brome et m est un nombre de 1 à 3, et les aluminoxanes ;
dans un rapport molaire AI/Ni inférieur ou égal à 8/1.

2. Composition selon la revendication 1, **caractérisée en ce que** les complexes de nickel mis en oeuvre ont pour formule générale LNiX₂ dans laquelle L est un ligand phosphonite bi- ou tridenté répondant à la formule générale : 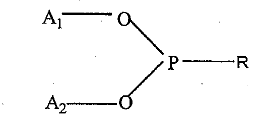 dans laquelle R représente un radical hydrocarboné monovalent, contenant jusqu'à 12 atomes de carbone et les radicaux A₁-O- et A₂-O-, identiques ou différents, sont choisis parmi les radicaux alkoxy ou aryloxy, et les radicaux alkoxy portant un hétérocycle azoté et X est un anion halogénure, un anion acétylacétonate ou un anion carboxylate.

3. Composition selon la revendication 2, **caractérisée en ce que**, dans le ligand phosphonite, R représente un radical alkyle, aryle, aralkyle, alkaryle, cycloalkyle ou aryle substitué.

4. Composition selon la revendication 2 ou 3, **caractérisée en ce que**, dans le ligand phosphonite, les radicaux A₁-O- et A₂-O-, identiques ou différents, sont des radicaux alkoxy-pyridine ou alkoxy-oxazoline, éventuellement substitués.

5. Composition selon la revendication 4, **caractérisée en ce que** chacun des radicaux A₁-O- et A₂-O- répond à l'une des formules générales : 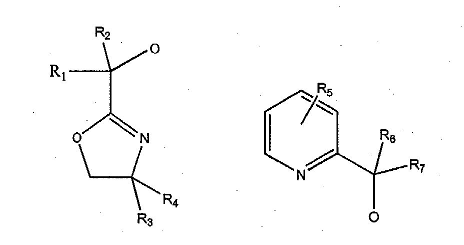 dans lesquelles les radicaux R₁, R₂, R₃, R₄, R₅, R₆ et R₇, identiques ou différents, sont choisis parmi l'atome d'hydrogène, les radicaux alkyles linéaires ou ramifiés, les radicaux aryles, aralkyles ou alkaryles comportant de 1 à 12 atomes de carbone, le radical R₅ étant sur l'une quelconque des positions libres du noyau aromatique.

6. Composition selon l'une des revendications 2 à 5, **caractérisée en ce que**, dans le complexe de nickel, X représente un anion Cl, Br, I, acétylacétonate, acétate ou trifluoroacétate.

7. Composition selon l'une des revendications 1 à 6, **caractérisée en ce que** le ligand phosphonite répond à l'une des formules générales : 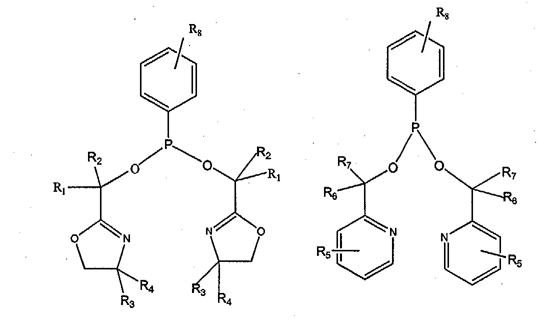 dans lesquelles les radicaux R₁, R₂, R₃, R₄, R₅, R₆, R₇ et R₈ sont identiques ou différents et choisis parmi l'atome d'hydrogène, les radicaux alkyles linéaires ou ramifiés les radicaux aryles, aralkyles et alkaryles comportant de 1 à 12 atomes de carbone, les radicaux R₅, R₈ et R₉ étant chacun sur l'une quelconque des positions libres des noyaux aromatiques.

8. Composition selon l'une des revendications 1 à 6, **caractérisée en ce que** le ligand phosphonite répond à l'une des formules générales : 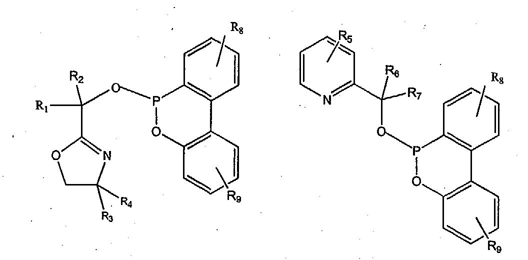 dans lesquelles les radicaux R₁, R₂, R₃, R₄, R₅, R₆, R₇, R₈, et R₉, identiques ou différents, sont choisis parmi l'atome d'hydrogène, les radicaux alkyles linéaires ou ramifiés et les radicaux aryles, aralkyles, alkaryles, comportant de 1 à 12 atomes de carbone, les radicaux R₅, et R₈ étant chacun sur l'une quelconque des positions libres des noyaux aromatiques.

9. Composition selon l'une des revendications 1 à 8, **caractérisée en ce que** le composé d'hydrocarbylaluminium est choisi parmi le dichloroéthylaluminium, le sesquichlorure d'éthylaluminium, le chlorodiéthylaluminium, le chlorodiisobutylaluminium, le triéthylaluminium, le tripropylaluminium, le triisobutylaluminium et le méthylaluminoxane.

10. Composition selon la revendication 9, **caractérisée en ce que** le composé d'hydrocarbylaluminium est le chlorodiéthylaluminium.

11. Composition selon l'une des revendications 1 à 10, **caractérisée en ce que** les composants du catalyseur sont mis en contact dans un solvant constitué par un hydrocarbure saturé, insaturé oléfinique ou aromatique.

12. Composition selon l'une des revendications 1 à 11, **caractérisée en ce que** la concentration du nickel dans la solution catalytique est de 1.10⁻⁵ à 0,1 mole/L.

13. Composition selon l'une des revendications 1 à 12, **caractérisée en ce que** le rapport molaire entre l'hydrocarbylaluminium et le complexe du nickel est de 1/1 à 8/1.

14. Procédé d'oligomérisation de l'éthylène utilisant une composition catalytique selon l'une des revendications 1 à 13.

15. Procédé selon la revendication 14, **caractérisé en ce que** la réaction d'oligomérisation de l'éthylène est effectuée sous une pression de 0,5 à 15 MPa et à une température de 20 à 180 °C.
